# EUROPEAN PATENT APPLICATION

(11) **EP 4 316 518 A1**
(43) Date of publication of application: **07.02.2024**
(21) Application number: 22778934.4
(22) Date of filing: 29.03.2022
(51) Int. Cl.: A61K 39/395, A61K 31/519, A61P 1/16, A61P 31/20

(54) **DRUG COMBINATION OF TOLL-LIKE RECEPTOR 7 AGONIST AND ANTI-PD-L1 ANTIBODY**

(30) Priority: 29.03.2021 CN 202110333959
(71) Applicant: CHIA TAI TIANQING PHARMACEUTICAL GROUP CO., LTD., Lianyungang, Jiangsu 222062 (CN)
(72) Inventor: XU, Zhongnan, Lianyungang, Jiangsu 222062 (CN); WANG, Shuna, Lianyungang, Jiangsu 222062 (CN); DENG, Jing, Lianyungang, Jiangsu 222062 (CN); HUO, Dandan, Lianyungang, Jiangsu 222062 (CN); WU, Ruonan, Lianyungang, Jiangsu 222062 (CN)
(74) Representative: Müller Schupfner & Partner Patent- und Rechtsanwaltspartnerschaft mbB
(86) International application number: PCT/CN2022/083661
(87) International publication number: WO 2022/206752

(57) **Abstract**

Provided is a drug combination of a Toll-like receptor 7 agonist and an anti-PD-L1 antibody. In particular, provided are a drug combination of a compound, which is represented by formula I and which acts as a TLR7 agonist, and an anti-PD-L1 antibody, and a medical use thereof.

## Description

### CROSS-REFERENCE TO RELATED APPLICATIONS

The present application claims priority and benefit to the Chinese Patent No. 202110333959.7 filed with the National Intellectual Property Administration, PRC on March 29, 2021, which is incorporated herein by reference in its entirety.

### TECHNICAL FIELD

The present application belongs to the field of medicinal chemistry, and relates to a pharmaceutical combination of a toll-like receptor 7 (TLR7) agonist and an anti-PD-L1 antibody, and specifically, to a pharmaceutical combination of a compound of formula I as a TLR7 agonist and an anti-PD-L1 antibody, and medicinal use thereof.

### BACKGROUND

According to World Health Organization statistics, about 257 million people worldwide are infected with the hepatitis B virus (HBV). If not well treated, patients with hepatitis will be confronted with long-term fatal diseases such as liver failure, cirrhosis and liver cancer.

Conventional therapies currently approved for the treatment of chronic hepatitis B include nucleosides (nucleotides) and interferons. Nucleosides (nucleotides), such as entecavir, can inhibit HBV DNA replication.

Toll-like receptors are expressed in a variety of immune cells. Toll-like receptors recognize highly conserved structural motifs: pathogen-associated molecular patterns (PAMPs) expressed by microbial pathogens or damage-associated molecular patterns (DAMPs) released by necrotic cells. Toll-like receptors are stimulated by corresponding PAMPs or DAMPs to induce a signaling cascade leading to the activation of transcription factors such as AP-1, NF-κB and interferon regulatory factors (impulse response functions). This results in a variety of cellular reactions, including the production of interferons, proinflammatory cytokines and effector cytokines, thereby producing immune responses. Thirteen toll-like receptors have been found in mammals to date. Toll-like receptors 1, 2, 4, 5, and 6 are mainly expressed on the cell surface while toll-like receptors 3, 7, 8, and 9 are expressed in endosomes. Different toll-like receptors recognize ligands derived from different pathogens. Toll-like receptor 7 (TLR7) is mainly expressed in plasmacytoid dendritic cells (pDCs) and induces interferon alpha (IFN-α) secretion by ligand recognition. Some TLR7 agonists have been reported, for example, imiquimod, resiquimod and GS-9620. WO2016023511 and WO2017076346, which are incorporated herein by reference in their entirety, disclose a class of new TLR7 agonists that demonstrate good bioactivity and selectivity.

Programmed death receptor ligand 1 (PD-L1) is a ligand for programmed death receptor 1 (PD-1). PD-1 is expressed mainly on lymphocytes and has two ligands, PD-L1 and PD-L2. PD-L2 is less common than PD-L1. PD-L1, also known as cluster of differentiation 274 (CD274) or B7 homolog 1 (B7-H1), is an approximately 40 kDa type 1 transmembrane protein encoded by the CD274 gene. The PD-1/PD-L1 pathway plays an important role in controlling immune responses. Dysfunctions in PD-1/PD-L1 signaling appear to be associated with the development and progression of diseases such as cancer and viral infections. Analysis of knockout animals has led to an understanding that PD-1/PD-L1 plays a major role in inducing and regulating peripheral tolerance. Thus, the therapeutic blocking of the PD-1/PD-L1 pathway would help overcome immune tolerance and treat cancer or infection as well as enhance immunity during vaccination (prophylactic or therapeutic).

Although patients with hepatitis B virus infections have many treatment options, there is still a need for more effective therapeutic agents for clinical use.

### SUMMARY

In one aspect, the present application provides a pharmaceutical combination comprising a toll-like receptor 7 agonist or a pharmaceutically acceptable salt thereof and an anti-PD-L1 antibody. Further, the pharmaceutical combination comprises entecavir or a pharmaceutically acceptable salt or solvate thereof.

In another aspect, the present application provides a kit for use in treating a hepatitis B virus infection, wherein comprising a pharmaceutical composition comprising a toll-like receptor 7 agonist and a pharmaceutical composition comprising an anti-PD-L1 antibody, the kit further comprising instructions for combined use for treating the hepatitis B virus infection. Further, the kit comprises entecavir or a pharmaceutically acceptable salt or solvate thereof.

In another aspect, the present application provides use of the pharmaceutical combination of the present application for preparing a medicament for treating a hepatitis B virus infection.

In another aspect, the present application provides a method for treating a hepatitis B virus infection, comprising administering to an individual in need thereof a therapeutically effective amount of the pharmaceutical combination of the present application.

In another aspect, the present application provides use of the pharmaceutical combination of the present application for treating a hepatitis B virus infection.

In another aspect, the present application provides a pharmaceutical combination for use in treating a hepatitis B virus infection.

### DETAILED DESCRIPTION OF INVENTION

In one aspect, the present application provides a pharmaceutical combination comprising a toll-like receptor 7 agonist or a pharmaceutically acceptable salt thereof and an anti-PD-L1 antibody.

In some embodiments, the pharmaceutical combination further comprises entecavir or a pharmaceutically acceptable salt or solvate thereof.

In some embodiments, the present application provides a pharmaceutical combination comprising: a toll-like receptor 7 agonist or a pharmaceutically acceptable salt thereof, an anti-PD-L1 antibody, and entecavir or a pharmaceutically acceptable salt or solvate thereof.

In some embodiments, the pharmaceutical combination is a fixed combination.

In some embodiments, the toll-like receptor 7 agonist or the pharmaceutically acceptable salt thereof and the anti-PD-L1 antibody in the fixed combination are present in the same pharmaceutical composition.

In some embodiments, the toll-like receptor 7 agonist or the pharmaceutically acceptable salt thereof, the anti-PD-L1 antibody, and entecavir or the pharmaceutically acceptable salt or solvate thereof, in the fixed combination are present in the same pharmaceutical composition.

In some embodiments, the pharmaceutical combination is a non-fixed combination.

In some embodiments, the toll-like receptor 7 agonist or the pharmaceutically acceptable salt thereof and the anti-PD-L1 antibody in the non-fixed combination are each in the form of a pharmaceutical composition.

In some embodiments, the toll-like receptor 7 agonist or the pharmaceutically acceptable salt thereof, the anti-PD-L1 antibody, and entecavir or the pharmaceutically acceptable salt or solvate thereof, in the non-fixed combination are each in the form of a pharmaceutical composition.

In some embodiments, the toll-like receptor 7 agonist or the pharmaceutically acceptable salt thereof, the anti-PD-L1 antibody, and/or entecavir or the pharmaceutically acceptable salt or solvate thereof, in the pharmaceutical combination are each in the form of a pharmaceutical composition.

In some embodiments, the toll-like receptor 7 agonist or the pharmaceutically acceptable salt thereof, the anti-PD-L1 antibody, and/or entecavir or the pharmaceutically acceptable salt or solvate thereof, in the pharmaceutical combination are packaged separately or together.

In some embodiments, the toll-like receptor 7 agonist or the pharmaceutically acceptable salt thereof, the anti-PD-L1 antibody, and/or entecavir or the pharmaceutically acceptable salt or solvate thereof, in the pharmaceutical combination are each packaged in separate kits, which further comprise instructions for combined use for treating a hepatitis B virus infection.

In some embodiments, the toll-like receptor 7 agonist or the pharmaceutically acceptable salt thereof, the anti-PD-L1 antibody, and/or entecavir or the pharmaceutically acceptable salt or solvate thereof, in the pharmaceutical combination are packaged in the same kit, which further comprises instructions for combined use for treating a hepatitis B virus infection.

In some embodiments, the present application provides a pharmaceutical combination comprising: a toll-like receptor 7 agonist or a pharmaceutically acceptable salt thereof and an anti-PD-L1 antibody in a weight ratio of 1:(100-1000). In some embodiments, the present application provides a pharmaceutical combination comprising a toll-like receptor 7 agonist or a pharmaceutically acceptable salt thereof, an anti-PD-L1 antibody, and entecavir or a pharmaceutically acceptable salt or solvate thereof in a weight ratio of 1:(100-1000):(0.5-5).

In some embodiments, the single dose ratio (on a weight basis) of the toll-like receptor 7 agonist or the pharmaceutically acceptable salt thereof to the anti-PD-L1 antibody in the pharmaceutical combination is 1:(100-1000). In some embodiments, the single dose ratio (on a weight basis) of the toll-like receptor 7 agonist or the pharmaceutically acceptable salt thereof to the anti-PD-L1 antibody to entecavir or the pharmaceutically acceptable salt or solvate thereof in the pharmaceutical combination is 1:(100-1000):(0.5-5).

In some embodiments, the present application provides a pharmaceutical combination comprising: a pharmaceutical composition comprising a single dose of 0.01 mg-10 mg of a toll-like receptor 7 agonist or a pharmaceutically acceptable salt thereof and a pharmaceutical composition comprising 200 mg-2400 mg of an anti-PD-L1 antibody. The pharmaceutical composition comprising the anti-PD-L1 antibody may be in a single dose or multiple doses.

In some embodiments, the present application provides a pharmaceutical combination comprising: a pharmaceutical composition comprising a single dose of 0.01 mg-10 mg of a toll-like receptor 7 agonist or a pharmaceutically acceptable salt thereof, a pharmaceutical composition comprising 200 mg-2400 mg of an anti-PD-L1 antibody, and a pharmaceutical composition comprising a single dose of 0.01 mg-10 mg of entecavir or a pharmaceutically acceptable salt or solvate thereof. The pharmaceutical composition comprising the anti-PD-L1 antibody is in a single dose or multiple doses.

In some embodiments, the present application provides a pharmaceutical combination comprising: a toll-like receptor 7 agonist or a pharmaceutically acceptable salt thereof and an anti-PD-L1 antibody in a weight ratio of 1:(10-200). In some embodiments, the present application provides a pharmaceutical combination comprising a toll-like receptor 7 agonist, an anti-PD-L1 antibody, and entecavir or a pharmaceutically acceptable salt or solvate thereof in a weight ratio of 1:(10-200):(1-10).

In some embodiments, the average daily dose ratio (on a weight basis) of the toll-like receptor 7 agonist or the pharmaceutically acceptable salt thereof to the anti-PD-L1 antibody in the pharmaceutical combination is 1:(10-200). In some embodiments, the average daily dose ratio (on a weight basis) of the toll-like receptor 7 agonist or the pharmaceutically acceptable salt thereof to the anti-PD-L1 antibody is selected from the group consisting of 1:10, 1:20, 1:30, 1:40, 1:50, 1:60, 1:70, 1:80, 1:90, 1:100, 1:110, 1:120, 1:130, 1:140, 1:150, 1:160, 1:170, 1:180, 1:190, 1:200, and a range formed by any of the above ratios. In some embodiments, the average daily dose ratio (on a weight basis) of the toll-like receptor 7 agonist or the pharmaceutically acceptable salt thereof to the anti-PD-L1 antibody is selected from the group consisting of 1:100, 1:101, 1:102, 1:103, 1:104, 1:105, 1:106, 1:107, 1:108, 1:109, 1:110, 1:111, 1:112, 1:113, 1:114, 1:115, 1:116, 1:117, 1:118, 1:119, 1:120, and a range formed by any of the above ratios. In some embodiments, the average daily dose ratio (on a weight basis) of the toll-like receptor 7 agonist or the pharmaceutically acceptable salt thereof to the anti-PD-L1 antibody is selected from the group consisting of 1:(100-120), 1:(102-118), 1:(104-116), and 1:(108-116).

In some of the embodiments, the average daily dose ratio (on a weight basis) of the toll-like receptor 7 agonist or the pharmaceutically acceptable salt thereof to entecavir or the pharmaceutically acceptable salt or solvate thereof in the pharmaceutical combination is selected from 1:(1-10). In some embodiments, the average daily dose ratio (on a weight basis) of the toll-like receptor 7 agonist or the pharmaceutically acceptable salt thereof to entecavir or the pharmaceutically acceptable salt or solvate thereof in the pharmaceutical combination is selected from the group consisting of 1:1.0, 1:1.1, 1:1.2, 1:1.3, 1:1.4, 1:1.5, 1:1.6, 1:1.7, 1:1.8, 1:1.9, 1:2.0, 1:2.1, 1:2.2, 1:2.3, 1:2.4, 1:2.5, 1:2.6, 1:2.7, 1:2.8, 1:2.9, 1:3.0, 1:3.1, 1:3.2, 1:3.3, 1:3.4, 1:3.5, 1:3.6, 1:3.7, 1:3.8, 1:3.9, 1:4.0, 1:4.1, 1:4.2, 1:4.3, 1:4.4, 1:4.5, 1:4.6, 1:4.7, 1:4.8, 1:4.9, 1:5.0, 1:5.1, 1:5.2, 1:5.3, 1:5.4, 1:5.5, 1:5.6, 1:5.7, 1:5.8, 1:5.9, 1:6.0, 1:6.1, 1:6.2, 1:6.3, 1:6.4, 1:6.5, 1:6.6, 1:6.7, 1:6.8, 1:6.9, 1:7.0, 1:7.1, 1:7.2, 1:7.3, 1:7.4, 1:7.5, 1:7.6, 1:7.7, 1:7.8, 1:7.9, 1:8.0, 1:8.1, 1:8.2, 1:8.3, 1:8.4, 1:8.5, 1:8.6, 1:8.7, 1:8.8, 1:8.9, 1:9.0, 1:9.1, 1:9.2, 1:9.3, 1:9.4, 1:9.5, 1:9.6, 1:9.7, 1:9.8, 1:9.9, 1:10.0, and a range formed by any of the above ratios. The average daily dose ratio (on a weight basis) of the toll-like receptor 7 agonist or the pharmaceutically acceptable salt thereof to entecavir or the pharmaceutically acceptable salt or solvate thereof in the pharmaceutical combination is selected from the group consisting of 1:(1.2-4.0), 1:(1.6-3.8), 1:(1.8-3.8), 1:(2.0-3.6), and 1:(2.5-3.5).

In some embodiments, the average daily dose ratio (on a weight basis) of the toll-like receptor 7 agonist or the pharmaceutically acceptable salt thereof to the anti-PD-L1 antibody to entecavir or the pharmaceutical salt or solvate thereof in the pharmaceutical combination is selected from the group consisting of 1:(10-200):(1-10), 1:(100-120):(1.2-4.0), 1:(102-118):(1.6-3.8), 1:(104-116):(1.8-3.8), and 1:(108-116): 1:(2.0-3.6).

In some embodiments, the present application provides a pharmaceutical combination comprising: a pharmaceutical composition comprising a single dose of 0.01 mg-10 mg of a toll-like receptor 7 agonist or a pharmaceutically acceptable salt thereof and a pharmaceutical composition comprising an anti-PD-L1 antibody at a concentration of 10-60 mg/mL. The pharmaceutical composition comprising the anti-PD-L1 antibody is in a single dose or multiple doses.

In some embodiments, the present application provides a pharmaceutical combination comprising: a pharmaceutical composition comprising a single dose of 0.01 mg-10 mg of a toll-like receptor 7 agonist or a pharmaceutically acceptable salt thereof, a pharmaceutical composition comprising an anti-PD-L1 antibody at a concentration of 10-60 mg/mL, and a pharmaceutical composition comprising a single dose of 0.01 mg-10 mg of entecavir or a pharmaceutically acceptable salt or solvate thereof. The pharmaceutical composition comprising the anti-PD-L1 antibody is in a single dose or multiple doses.

In another aspect, the present application provides a kit for use in treating a hepatitis B virus infection, wherein comprising a pharmaceutical composition comprising a toll-like receptor 7 agonist or a pharmaceutically acceptable salt thereof and a pharmaceutical composition comprising an anti-PD-L1 antibody, the kit further comprising instructions for combined use for treating the hepatitis B virus infection.

In some embodiments, the kit further comprises a pharmaceutical composition comprising entecavir or a pharmaceutically acceptable salt or solvate thereof.

In some embodiments, the present application provides a kit comprising a pharmaceutical composition comprising a toll-like receptor 7 agonist or a pharmaceutically acceptable salt thereof, a pharmaceutical composition comprising an anti-PD-L1 antibody, and a pharmaceutical composition comprising entecavir or a pharmaceutically acceptable salt or solvate thereof, the kit further comprising instructions for combined use for treating a hepatitis B virus infection.

In another aspect, the present application provides use of the pharmaceutical combination of the present application for preparing a medicament for treating a hepatitis B virus infection.

In another aspect, the present application provides a method for treating a hepatitis B virus infection, comprising administering to an individual in need thereof a therapeutically effective amount of the pharmaceutical combination of the present application.

In another aspect, the present application provides use of the pharmaceutical combination of the present application for treating a hepatitis B virus infection.

In another aspect, the present application provides a pharmaceutical combination for use in treating a hepatitis B virus infection.

### Toll-Like Receptor 7 Agonist or Pharmaceutically Acceptable Salt Thereof or Pharmaceutical Composition Thereof

In some embodiments, the toll-like receptor 7 agonist or the pharmaceutically acceptable salt thereof is selected from a compound of formula **I** or a pharmaceutically acceptable salt thereof:

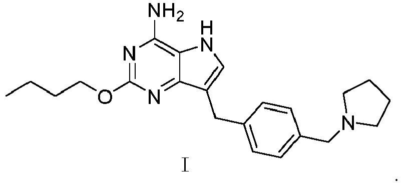

The compound of formula **I** belongs to the prior art and has the chemical name 2-butoxy-7-(4-(pyrrolidin-1-ylmethyl)benzyl)-5*H*-pyrrolo[3,2-*d*]pyrimidin-4-amine, of which the preparation and chemical properties can be found in WO2016023511.

In some embodiments, the pharmaceutically acceptable salt of the compound of formula **I** is selected from the group consisting of a maleate and a fumarate.

In some embodiments, the pharmaceutical composition of the compound of formula **I** or the pharmaceutically acceptable salt thereof is selected from a solid pharmaceutical composition, preferably from the group consisting of a tablet and a capsule.

In some embodiments, the toll-like receptor 7 agonist or the pharmaceutically acceptable salt thereof is in the form of a pharmaceutical composition in a single dose of 0.01 mg-10 mg, preferably a pharmaceutical composition in a single dose of 0.01 mg, 0.02 mg, 0.05 mg, 0.08 mg, 0.1 mg, 0.2 mg, 0.3 mg, 0.4 mg, 0.5 mg, 0.6 mg, 0.7 mg, 0.8 mg, 0.9 mg, 1.0 mg, 1.1 mg, 1.2 mg, 1.3 mg, 1.4 mg, 1.5 mg, 1.6 mg, 1.7 mg, 1.8 mg, 1.9 mg, 2.0 mg, 2.1 mg, 2.2 mg, 2.3 mg, 2.4 mg, 2.5 mg, 2.6 mg, 2.7 mg, 2.8 mg, 2.9 mg, 3.0 mg, 3.1 mg, 3.2 mg, 3.3 mg, 3.4 mg, 3.5 mg, 3.6 mg, 3.7 mg, 3.8 mg, 3.9 mg, 4.0 mg, 4.1 mg, 4.2 mg, 4.3 mg, 4.4 mg, 4.5 mg, 4.6 mg, 4.7 mg, 4.8 mg, 4.9 mg, 5.0 mg, 5.1 mg, 5.2 mg, 5.3 mg, 5.4 mg, 5.5 mg, 5.6 mg, 5.7 mg, 5.8 mg, 5.9 mg, 6.0 mg, 6.1 mg, 6.2 mg, 6.3 mg, 6.4 mg, 6.5 mg, 6.6 mg, 6.7 mg, 6.8 mg, 6.9 mg, 7.0 mg, 7.1 mg, 7.2 mg, 7.3 mg, 7.4 mg, 7.5 mg, 7.6 mg, 7.7 mg, 7.8 mg, 7.9 mg, 8.0 mg, 8.1 mg, 8.2 mg, 8.3 mg, 8.4 mg, 8.5 mg, 8.6 mg, 8.7 mg, 8.8 mg, 8.9 mg, 9.0 mg, 9.1 mg, 9.2 mg, 9.3 mg, 9.4 mg, 9.5 mg, 9.6 mg, 9.7 mg, 9.8 mg, 9.9 mg, 10.0 mg, or a range formed by any of the above values.

In some embodiments, the toll-like receptor 7 agonist or the pharmaceutically acceptable salt thereof is in the form of a pharmaceutical composition in a single dose of 0.1 mg-1.0 mg, preferably a pharmaceutical composition in a single dose of 0.2 mg or 0.5 mg.

In some embodiments, the toll-like receptor 7 agonist or the pharmaceutically acceptable salt thereof may be selected from the group consisting of imiquimod, GSK-2245035, resiquimod, vesatolimod (GS-9620), telratolimod, TMX-202, DSP-0509, RG-7854, SHR2150, and loxoribine.

### Anti-PD-L1 Antibody or Pharmaceutical Composition Thereof

In some embodiments, the anti-PD-L1 antibody is an anti-PD-L1 antibody disclosed in WO2016022630 or CN107001463A.

In some embodiments, the anti-PD-L1 antibody comprises the following amino acid sequences: a heavy chain complementarity determining region (CDR) 1 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology with an amino acid sequence set forth in SEQ ID NO: 1 or SEQ ID NO: 4; a heavy chain CDR2 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology with an amino acid sequence set forth in SEQ ID NO: 2 or SEQ ID NO: 5; a heavy chain CDR3 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology with an amino acid sequence set forth in SEQ ID NO: 3 or SEQ ID NO: 6; a light chain CDR1 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology with an amino acid sequence set forth in SEQ ID NO: 7 or SEQ ID NO: 10; a light chain CDR2 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology with an amino acid sequence set forth in SEQ ID NO: 8 or SEQ ID NO: 11; and a light chain CDR3 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology with an amino acid sequence set forth in SEQ ID NO: 9 or SEQ ID NO: 12.

In some embodiments, the anti-PD-L1 antibody comprises the following amino acid sequences: a heavy chain CDR1 region selected from the group consisting of SEQ ID NO: 1 and SEQ ID NO: 4; a heavy chain CDR2 region selected from the group consisting of SEQ ID NO: 2 and SEQ ID NO: 5; a heavy chain CDR3 region selected from the group consisting of SEQ ID NO: 3 and SEQ ID NO: 6; a light chain CDR1 region selected from the group consisting of SEQ ID NO: 7 and SEQ ID NO: 10; a light chain CDR2 region selected from the group consisting of SEQ ID NO: 8 and SEQ ID NO: 11; and a light chain CDR3 region selected from the group consisting of SEQ ID NO: 9 and SEQ ID NO: 12.

In some embodiments, the anti-PD-L1 antibody described herein comprises: a heavy chain CDR1 region having an amino acid sequence set forth in SEQ ID NO: 1; a heavy chain CDR2 region having an amino acid sequence set forth in SEQ ID NO: 2; a heavy chain CDR3 region having an amino acid sequence set forth in SEQ ID NO: 3; a light chain CDR1 region having an amino acid sequence set forth in SEQ ID NO: 7; a light chain CDR2 region having an amino acid sequence set forth in SEQ ID NO: 8; and a light chain CDR3 region having an amino acid sequence set forth in SEQ ID NO: 9.

In some embodiments, the anti-PD-L1 antibody described herein comprises: a heavy chain CDR1 region having an amino acid sequence set forth in SEQ ID NO: 4; a heavy chain CDR2 region having an amino acid sequence set forth in SEQ ID NO: 5; a heavy chain CDR3 region having an amino acid sequence set forth in SEQ ID NO: 6; a light chain CDR1 region having an amino acid sequence set forth in SEQ ID NO: 10; a light chain CDR2 region having an amino acid sequence set forth in SEQ ID NO: 11; and a light chain CDR3 region having an amino acid sequence set forth in SEQ ID NO: 12.

The CDR regions described herein and the variants thereof described above are capable of specifically recognizing and binding to PD-L1, thereby effectively blocking the signaling between PD-L1 and PD-1.

In some embodiments, the anti-PD-L1 antibody described herein comprises the following amino acid sequences: a heavy chain variable region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology with an amino acid sequence set forth in SEQ ID NO: 13 or SEQ ID NO: 14; and a light chain variable region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology with an amino acid sequence set forth in SEQ ID NO: 15 or SEQ ID NO: 16.

In some embodiments, the anti-PD-L1 antibody comprises the following amino acid sequences: a heavy chain variable region set forth in SEQ ID NO: 13 and a light chain variable region set forth in SEQ ID NO: 15.

In some embodiments, the anti-PD-L1 antibody comprises the following amino acid sequences: a heavy chain variable region set forth in SEQ ID NO: 14 and a light chain variable region set forth in SEQ ID NO: 16.

In some embodiments, the anti-PD-L1 antibody comprises the following amino acid sequences: a heavy chain amino acid sequence set forth in SEQ ID NO: 17 and a light chain amino acid sequence set forth in SEQ ID NO: 18.

In some embodiments, the anti-PD-L1 antibody comprises the following amino acid sequences: a heavy chain amino acid sequence set forth in SEQ ID NO: 19 and a light chain amino acid sequence set forth in SEQ ID NO: 20.

In some embodiments, the anti-PD-L1 antibody comprises the following amino acid sequences: a heavy chain amino acid sequence set forth in SEQ ID NO: 21 and a light chain amino acid sequence set forth in SEQ ID NO: 18.

In some embodiments, the anti-PD-L1 antibody is a humanized antibody. In some embodiments, the anti-PD-L1 antibody is an anti-PD-L1 mAb.

In some embodiments, the anti-PD-L1 antibody provided by the present application comprises one or more conservatively substituted variants selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, and SEQ ID NO: 21. The humanized anti-PD-L1 mAb comprising the conservatively substituted variants retains the ability to specifically recognize and bind to PD-L1.

In some embodiments, the anti-PD-L1 antibody may be an IgG1 or IgG4 antibody.

In some embodiments, the anti-PD-L1 antibody is an IgG1 antibody. In some embodiments, the anti-PD-L1 antibody is a glycosylated IgG1 antibody.

In some embodiments, the anti-PD-L1 antibody comprises a heavy chain CDR region selected from the group consisting of heavy chain CDR regions of antibodies 13C5 and 5G11; and a light chain CDR region selected from the group consisting of light chain CDR regions of antibodies 13C5 or 5G11. In one embodiment, the anti-PD-L1 antibody described herein comprises: a heavy chain variable region selected from the group consisting of heavy chain variable regions of chimeric antibodies ch5G11-hIgG1, ch5G11-hIgG4, ch13C5-hIgG1, and ch13C5-hIgG4; and a light chain variable region selected from the group consisting of light chain variable regions of chimeric antibodies ch5G11-hIgG1, ch5G11-hIgG4, ch13C5-hIgG1, and ch13C5-hIgG4. In one embodiment, the anti-PD-L1 antibody described herein comprises: a heavy chain variable region selected from the group consisting of heavy chain variable regions of humanized antibodies hu13C5-hIgG1, hu13C5-hIgG4, hu5G11-hIgG1, and hu5G11-hIgG4; and a light chain variable region selected from the group consisting of light chain variable regions of humanized antibodies hu13C5-hIgG1, hu13C5-hIgG4, hu5G11-hIgG1, and hu5G11-hIgG4. For example, reference can be made to the records of the patent WO2016022630 or CN107001463A: 13C5, ch13C5-hIgG1, ch13C5-hIgG4, hu13C5-hIgG1, or hu13C5-hIgG4 comprises a heavy chain CDR1 sequence of SYGMS (SEQ ID NO: 4), a heavy chain CDR2 sequence of SISSGGSTYYPDSVKG (SEQ ID NO: 5), a heavy chain CDR3 sequence of GYDSGFAY (SEQ ID NO: 6), a light chain CDR1 sequence of ASQSVSTSSSSFMH (SEQ ID NO: 10), a light chain CDR2 sequence of YASNLES (SEQ ID NO: 11), and a light chain CDR3 sequence of QHSWEIPYT (SEQ ID NO: 12); 5G11, ch5G11-hIgG1, ch5G11-hIgG4, hu5G11-hIgG1, or hu5G11-hIgG4 comprises a heavy chain CDR1 sequence of TYGVH (SEQ ID NO: 1), a heavy chain CDR2 sequence of VIWRGVTTDYNAAFMS (SEQ ID NO: 2), a heavy chain CDR3 sequence of LGFYAMDY (SEQ ID NO: 3), a light chain CDR1 sequence of KASQSVSNDVA (SEQ ID NO: 7), a light chain CDR2 sequence of YAANRYT (SEQ ID NO: 8), and a light chain CDR3 sequence of QQDYTSPYT (SEQ ID NO: 9).

In some embodiments, the anti-PD-L1 antibody may be selected from one or more anti-PD-L1 antibodies. As used herein, the term "more" refers to more than one, for example, two, three, four, five or more. For example, in some embodiments, the anti-PD-L1 antibody is selected from the group consisting of anti-PD-L1 antibodies as follows: an anti-PD-L1 antibody comprising a heavy chain variable region set forth in SEQ ID NO: 13 and a light chain variable region set forth in SEQ ID NO: 15, or an anti-PD-L1 antibody comprising a heavy chain variable region set forth in SEQ ID NO: 14 and a light chain variable region set forth in SEQ ID NO: 16, or a combination of the above. As another example, the anti-PD-L1 antibody is selected from the group consisting of anti-PD-L1 antibodies as follows: an anti-PD-L1 antibody comprising a heavy chain amino acid sequence set forth in SEQ ID NO: 17 and a light chain amino acid sequence set forth in SEQ ID NO: 18, or an anti-PD-L1 antibody comprising a heavy chain amino acid sequence set forth in SEQ ID NO: 19 and a light chain amino acid sequence set forth in SEQ ID NO: 20, or an anti-PD-L1 antibody comprising a heavy chain amino acid sequence set forth in SEQ ID NO: 21 and a light chain amino acid sequence set forth in SEQ ID NO: 18, or a combination of any of the above. In some embodiments, the anti-PD-L1 antibody is in the form of a pharmaceutical composition comprising a single dose of 200 mg-600 mg of the anti-PD-L1 antibody, preferably a single dose of 200 mg, 300 mg, 400 mg, 500 mg, or 600 mg of the anti-PD-L1 antibody.

In some embodiments, the anti-PD-L1 antibody is in the form of a pharmaceutical composition comprising a single dose of 20 mg-200 mg of the anti-PD-L1 antibody, preferably a single dose of 20 mg, 50 mg, 80 mg, 100 mg, 150 mg, or 200 mg of the anti-PD-L1 antibody.

In some embodiments, the total dose of the pharmaceutical composition of the anti-PD-L1 antibody is 200 mg-2400 mg. In some embodiments, the total dose of the pharmaceutical composition of the anti-PD-L1 antibody is selected from the group consisting of 200 mg, 300 mg, 400 mg, 500 mg, 600 mg, 900 mg, 1200 mg, 1500 mg, 1800 mg, 2100 mg, 2400 mg, and a range formed by any of the above values. In some embodiments, the total dose of the pharmaceutical composition of the anti-PD-L1 antibody is preferably 200 mg-800 mg, 200 mg-1200 mg, 600 mg-2100 mg, or 900 mg-1500 mg.

In some embodiments, the pharmaceutical composition of the anti-PD-L1 antibody further comprises one or more of a buffer, an isotonicity modifier, a stabilizer, and/or a surfactant. In particular, the pharmaceutical composition of the anti-PD-L1 antibody comprises 1-150 mg/mL anti-PD-L1 antibody (e.g., mAb), 3-50 mM buffer, 2-150 mg/mL isotonicity modifier/stabilizer and 0.01-0.8 mg/mL surfactant, and has a pH of about 4.5-6.8.

In some embodiments, in the pharmaceutical composition of the anti-PD-L1 antibody, the concentration (w/v) of the anti-PD-L1 mAb is about 5-150 mg/mL, preferably about 10-60 mg/mL, and more preferably about 10-30 mg/mL. In some specific embodiments, the concentration (w/v) of the anti-PD-L1 mAb is about 10 mg/mL, about 20 mg/mL, about 30 mg/mL, about 40 mg/mL, about 50 mg/mL, about 60 mg/mL, about 70 mg/mL, about 80 mg/mL, about 90 mg/mL, about 100 mg/mL, about 110 mg/mL, or about 120 mg/mL; preferably about 10 mg/mL, about 20 mg/mL, about 30 mg/mL, about 40 mg/mL, about 50 mg/mL, or about 60 mg/mL; and more preferably about 10 mg/mL, about 20 mg/mL, or about 30 mg/mL. In some embodiments, the concentration (w/v) of the anti-PD-L1 mAb is about 10 mg/mL. In other embodiments, the concentration (w/v) of the anti-PD-L1 mAb is about 30 mg/mL. In other embodiments, the concentration (w/v) of the anti-PD-L1 mAb is about 60 mg/mL.

In some embodiments, the buffer is a histidine salt buffer. The concentration of the histidine salt buffer is about 5-30 mM, preferably about 10-25 mM, more preferably about 10-20 mM, and most preferably about 10-15 mM. In some specific embodiments, the concentration of the histidine salt buffer is about 5 mM, about 10 mM, about 15 mM, about 20 mM, about 25 mM, or about 30 mM. In some embodiments, the concentration of the histidine salt buffer is about 10 mM. In other embodiments, the concentration of the histidine salt buffer is about 15 mM. In other embodiments, the concentration of the histidine salt buffer is about 20 mM. The histidine salt buffer comprises histidine and hydrochloric acid.

In some embodiments, the isotonicity modifier/stabilizer is sucrose of about 20-150 mg/mL (w/v), preferably sucrose of about 40-100 mg/mL (w/v), and more preferably sucrose of about 60-80 mg/mL (w/v). In some specific embodiments, the concentration of the sucrose is about 40 mg/mL, 50 mg/mL, 60 mg/mL, 70 mg/mL, 80 mg/mL, 90 mg/mL, or 100 mg/mL. In some specific embodiments, the concentration of the sucrose is about 60 mg/mL. In some specific embodiments, the concentration of the sucrose is about 70 mg/mL. In some specific embodiments, the concentration of the sucrose is about 80 mg/mL. In some specific embodiments, the concentration of the sucrose is about 90 mg/mL.

In some embodiments, the surfactant is selected from the group consisting of polysorbate 80, polysorbate 20, and poloxamer 188; preferably polysorbate 80 and polysorbate 20; and more preferably polysorbate 80. In some embodiments, the concentration (w/v) of the surfactant is about 0.05-0.6 mg/mL, preferably about 0.1-0.4 mg/mL, and more preferably about 0.2-0.3 mg/mL.

In some embodiments, the surfactant is polysorbate 80 or polysorbate 20 of about 0.01-0.8 mg/mL (w/v). In some specific embodiments, the surfactant is polysorbate 80 of about 0.05-0.6 mg/mL, preferably polysorbate 80 of about 0.1-0.4 mg/mL, more preferably polysorbate 80 of about 0.2-0.3 mg/mL, and most preferably about polysorbate 80 of 0.2 mg/mL. In some embodiments, the concentration of polysorbate 80 in the pharmaceutical composition is about 0.1 mg/mL, 0.2 mg/mL, 0.3 mg/mL, 0.4 mg/mL, 0.5 mg/mL, or 0.6 mg/mL; preferably, the concentration of polysorbate 80 in the pharmaceutical composition is about 0.2 mg/mL, 0.3 mg/mL, 0.4 mg/mL, or 0.5 mg/mL; more preferably, the concentration of polysorbate 80 in the pharmaceutical composition is about 0.2 mg/mL, 0.3 mg/mL, or 0.4 mg/mL; most preferably, the concentration of polysorbate 80 in the pharmaceutical composition is about 0.2 mg/mL. In some embodiments, the concentration of polysorbate 80 in the pharmaceutical composition is about 0.1 mg/mL. In other embodiments, the concentration of polysorbate 80 in the pharmaceutical composition is about 0.2 mg/mL. In some embodiments, the concentration of polysorbate 80 in the pharmaceutical composition is about 0.3 mg/mL. In other embodiments, the concentration of polysorbate 80 in the pharmaceutical composition is about 0.4 mg/mL. In some embodiments, the concentration of polysorbate 80 in the pharmaceutical composition is about 0.5 mg/mL.

In some embodiments, an aqueous solution of the pharmaceutical composition has a pH of 4.0-6.8, preferably 4.5-6.5, more preferably 5.5-6.0, and most preferably 5.5. In some embodiments, an aqueous solution of the pharmaceutical composition has a pH of about 4.5, about 4.8, about 5.0, about 5.2, about 5.4, about 5.5, about 5.6, about 5.8 or about 6.0, preferably about 5.0, about 5.2, about 5.4, about 5.5, or about 5.6, and more preferably about 5.5. In some embodiments, an aqueous solution of the pharmaceutical composition has a pH of about 5.0. In some embodiments, an aqueous solution of the pharmaceutical composition has a pH of about 5.2. In some embodiments, an aqueous solution of the pharmaceutical composition has a pH of about 5.4. In some embodiments, an aqueous solution of the pharmaceutical composition has a pH of about 5.5. In some embodiments, an aqueous solution of the pharmaceutical composition has a pH of about 5.6. In some embodiments, an aqueous solution of the pharmaceutical composition has a pH of about 5.8. In some embodiments, an aqueous solution of the pharmaceutical composition has a pH of about 6.0.

In some specific embodiments of the present application, the pharmaceutical composition comprises: (a) an anti-PD-L1 antibody at a concentration (w/v) of about 5-150 mg/mL, preferably about 10-60 mg/mL, and more preferably about 10-30 mg/mL, (b) sucrose at a concentration (w/v) of about 20-150 mg/mL, preferably about 40-100 mg/mL, and more preferably about 60-80 mg/mL, (c) polysorbate 80 at a concentration (w/v) of about 0.05-0.6 mg/mL, preferably about 0.1-0.4 mg/mL, and more preferably about 0.2-0.3 mg/mL, (d) histidine at a molar concentration of about 5-30 mM, preferably about 10-25 mM, more preferably about 10-20 mM, and most preferably about 10-15 mM, and (e) optionally a suitable amount of hydrochloric acid for adjusting the pH of the composition to about 4.0-6.8, preferably 4.5-6.5, more preferably 5.5-6.0, and most preferably 5.5.

In some specific embodiments of the present application, the pharmaceutical composition comprises: (a) an anti-PD-L1 antibody at a concentration (w/v) of about 20 mg/mL, (b) sucrose at a concentration (w/v) of about 70 mg/mL, (c) polysorbate 80 at a concentration (w/v) of about 0.1 mg/mL, (d) histidine at a molar concentration of about 20 mM, and (e) optionally a suitable amount of hydrochloric acid for adjusting the pH of the composition to about 5.0.

In one specific embodiment of the present application, the pharmaceutical composition comprises: (a) an anti-PD-L1 mAb at a concentration (w/v) of about 20 mg/mL, (b) sucrose at a concentration (w/v) of about 70 mg/mL, (c) polysorbate 80 at a concentration (w/v) of about 0.1 mg/mL, (d) histidine at a molar concentration of about 20 mM, and (e) optionally a suitable amount of hydrochloric acid for adjusting the pH of the composition to about 5.0.

In one specific embodiment of the present application, the pharmaceutical composition comprises: (a) an anti-PD-L1 antibody at a concentration (w/v) of about 10 mg/mL, (b) sucrose at a concentration (w/v) of about 80 mg/mL, (c) polysorbate 80 at a concentration (w/v) of about 0.2 mg/mL, (d) histidine at a molar concentration of about 10 mM, and (e) optionally a suitable amount of hydrochloric acid for adjusting the pH of the composition to about 5.5.

In another specific embodiment of the present application, the pharmaceutical composition comprises: (a) an anti-PD-L1 antibody at a concentration (w/v) of about 50 mg/mL, (b) sucrose at a concentration (w/v) of about 80 mg/mL, (c) polysorbate 80 at a concentration (w/v) of about 0.3 mg/mL, (d) histidine at a molar concentration of about 10 mM, and (e) optionally a suitable amount of hydrochloric acid for adjusting the pH of the composition to about 5.5.

In another specific embodiment of the present application, the pharmaceutical composition comprises: (a) an anti-PD-L1 antibody at a concentration (w/v) of about 100 mg/mL, (b) sucrose at a concentration (w/v) of about 80 mg/mL, (c) polysorbate 80 at a concentration (w/v) of about 0.5 mg/mL, (d) histidine at a molar concentration of about 10 mM, and (e) optionally a suitable amount of hydrochloric acid for adjusting the pH of the composition to about 5.5.

In one specific embodiment of the present application, the pharmaceutical composition comprises: (a) an anti-PD-L1 antibody at a concentration (w/v) of about 30 mg/mL, (b) sucrose at a concentration (w/v) of about 80 mg/mL, (c) polysorbate 80 at a concentration (w/v) of about 0.2 mg/mL, (d) histidine at a molar concentration of about 10 mM, and (e) optionally a suitable amount of about hydrochloric acid for adjusting the pH of the composition to about 5.5.

In another specific embodiment of the present application, the pharmaceutical composition comprises: (a) an anti-PD-L1 antibody at a concentration (w/v) of about 60 mg/mL, (b) sucrose at a concentration (w/v) of about 80 mg/mL, (c) polysorbate 80 at a concentration (w/v) of about 0.2 mg/mL, (d) histidine at a molar concentration of about 10 mM, and (e) optionally a suitable amount of hydrochloric acid for adjusting the pH of the composition to about 5.5.

In one specific embodiment of the present application, the pharmaceutical composition comprises: (a) an anti-PD-L1 antibody at a concentration (w/v) of about 10 mg/mL, (b) sucrose at a concentration (w/v) of about 70 mg/mL, (c) polysorbate 80 at a concentration (w/v) of about 0.4 mg/mL, (d) histidine at a molar concentration of about 20 mM, and (e) optionally a suitable amount of acetic acid for adjusting the pH of the composition to about 6.5.

In one specific embodiment of the present application, the pharmaceutical composition comprises: (a) an anti-PD-L1 mAb at a concentration (w/v) of about 10 mg/mL, (b) sucrose at a concentration (w/v) of about 80 mg/mL, (c) polysorbate 80 at a concentration (w/v) of about 0.2 mg/mL, (d) histidine at a molar concentration of about 20 mM, and (e) optionally a suitable amount of hydrochloric acid for adjusting the pH of the composition to about 5.5.

In another specific embodiment of the present application, the pharmaceutical composition is a water-soluble injection, including but not limited to unlyophilized water-soluble formulations or water-soluble formulations obtained by reconstitution of lyophilized powders. In other embodiments, the pharmaceutical composition is a lyophilized formulation. The lyophilized formulation refers to a formulation prepared by subjecting an aqueous solution to a lyophilization process, e.g., in which a substance is first frozen, and then the amount of a solvent is reduced by sublimation (primary drying process) and then by desorption (secondary drying process) until the amount of the solvent is reduced to a value that no longer supports a biological activity or a chemical reaction. The lyophilized formulation of the present application can also be dried by other methods known in the art, such as spray drying and bubble drying.

### Entecavir or Pharmaceutically Acceptable Salt or Solvate Thereof, or Pharmaceutical Composition Thereof

As used herein, entecavir has the chemical name 2-amino-9-[(1*S*,3*R*,4*S*)-4-hydroxy-3-hydroxymethyl-2-methylenecyclopentyl]-1,9-dihydro-6*H*-purin-6-one and the following structural formula:

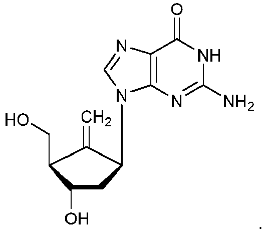

In some embodiments, the pharmaceutically acceptable salt of entecavir is selected from a maleate. In some embodiments, the pharmaceutically acceptable salt of entecavir is selected from a monomaleate.

In some embodiments, the entecavir solvate is selected from an entecavir hydrate. In some embodiments, the entecavir hydrate is selected from the group consisting of entecavir 0.5-2 hydrate. In some embodiments, the entecavir hydrate is selected from entecavir monohydrate.

In some embodiments, entecavir or the pharmaceutically acceptable salt or solvate thereof is selected from the group consisting of entecavir monomaelate, entecavir monohydrate, and entecavir monomaelate monohydrate.

In some embodiments, entecavir or the pharmaceutically acceptable salt or solvate thereof is in the form of a pharmaceutical composition; preferably, the pharmaceutical composition is selected from a solid pharmaceutical composition; the solid pharmaceutical composition is preferably selected from the group consisting of a tablet and a capsule.

In some embodiments, the pharmaceutical composition of entecavir or the pharmaceutically acceptable salt or solvate thereof is selected from a pharmaceutical composition in a single dose of 0.01 mg-5 mg, preferably from a pharmaceutical composition in a single dose of 0.01 mg, 0.02 mg, 0.05 mg, 0.08 mg, 0.1 mg, 0.2 mg, 0.3 mg, 0.4 mg, 0.5 mg, 0.6 mg, 0.7 mg, 0.8 mg, 0.9 mg, 1.0 mg, 1.1 mg, 1.2 mg, 1.3 mg, 1.4 mg, 1.5 mg, 1.6 mg, 1.7 mg, 1.8 mg, 1.9 mg, 2.0 mg, 2.1 mg, 2.2 mg, 2.3 mg, 2.4 mg, 2.5 mg, 2.6 mg, 2.7 mg, 2.8 mg, 2.9 mg, 3.0 mg, 3.1 mg, 3.2 mg, 3.3 mg, 3.4 mg, 3.5 mg, 3.6 mg, 3.7 mg, 3.8 mg, 3.9 mg, 4.0 mg, 4.1 mg, 4.2 mg, 4.3 mg, 4.4 mg, 4.5 mg, 4.6 mg, 4.7 mg, 4.8 mg, 4.9 mg, 5.0 mg, or a range formed by any of the above values.

In some embodiments, the pharmaceutical composition of entecavir or the pharmaceutically acceptable salt or solvate thereof is selected from a pharmaceutical composition in a single dose of 0.5 mg.

As used herein, entecavir or the pharmaceutically acceptable salt or solvate thereof, or the pharmaceutical composition thereof may be selected from a commercially available product.

### Administration/Treatment Regimen of Pharmaceutical Combination

In some embodiments, effective amounts of the toll-like receptor 7 agonist or the pharmaceutically acceptable salt thereof, the anti-PD-L1 antibody, and entecavir or the pharmaceutically acceptable salt or solvate thereof may be administered simultaneously, sequentially, or at intervals to an individual in need thereof.

In some embodiments, effective amounts of the toll-like receptor 7 agonist or the pharmaceutically acceptable salt thereof and entecavir or the pharmaceutically acceptable salt or solvate thereof may be administered simultaneously to an individual in need thereof, and an effective amount of the anti-PD-L1 antibody may be administered at intervals.

In some embodiments, effective amounts of the toll-like receptor 7 agonist or the pharmaceutically acceptable salt thereof, the anti-PD-L1 antibody, and entecavir or the pharmaceutically acceptable salt or solvate thereof may be administered to an individual in need thereof by following the same regimen or different regimens.

In some embodiments, the toll-like receptor 7 agonist or the pharmaceutically acceptable salt thereof may be administered thrice every day, twice every day, once every day, once every two days, once every three days, once every four days, once every five days, once every six days, once every week, once every two weeks, or once every three weeks.

In some embodiments, the toll-like receptor 7 agonist or the pharmaceutically acceptable salt thereof is administered at a dose of 0.1-10.0 mg each time, preferably a dose of 0.1 mg, 0.2 mg, 0.3 mg, 0.4 mg, 0.5 mg, 0.6 mg, 0.7 mg, 0.8 mg, 0.9 mg, 1.0 mg, 1.1 mg, 1.2 mg, 1.3 mg, 1.4 mg, 1.5 mg, 1.6 mg, 1.7 mg, 1.8 mg, 1.9 mg, 2.0 mg, 2.1 mg, 2.2 mg, 2.3 mg, 2.4 mg, 2.5 mg, 2.6 mg, 2.7 mg, 2.8 mg, 2.9 mg, 3.0 mg, 3.1 mg, 3.2 mg, 3.3 mg, 3.4 mg, 3.5 mg, 3.6 mg, 3.7 mg, 3.8 mg, 3.9 mg, 4.0 mg, 4.1 mg, 4.2 mg, 4.3 mg, 4.4 mg, 4.5 mg, 4.6 mg, 4.7 mg, 4.8 mg, 4.9 mg, 5.0 mg, 5.1 mg, 5.2 mg, 5.3 mg, 5.4 mg, 5.5 mg, 5.6 mg, 5.7 mg, 5.8 mg, 5.9 mg, 6.0 mg, 6.1 mg, 6.2 mg, 6.3 mg, 6.4 mg, 6.5 mg, 6.6 mg, 6.7 mg, 6.8 mg, 6.9 mg, 7.0 mg, 7.1 mg, 7.2 mg, 7.3 mg, 7.4 mg, 7.5 mg, 7.6 mg, 7.7 mg, 7.8 mg, 7.9 mg, 8.0 mg, 8.1 mg, 8.2 mg, 8.3 mg, 8.4 mg, 8.5 mg, 8.6 mg, 8.7 mg, 8.8 mg, 8.9 mg, 9.0 mg, 9.1 mg, 9.2 mg, 9.3 mg, 9.4 mg, 9.5 mg, 9.6 mg, 9.7 mg, 9.8 mg, 9.9 mg, 10.0 mg, or a range formed by any of the above values. In some embodiments, the toll-like receptor 7 agonist or the pharmaceutically acceptable salt thereof is administered at a dose of 0.2-5.0 mg, 0.4-4.0 mg, 0.5-3.0 mg, 0.6-2.6 mg, 0.8-2.2 mg, 0.8-1.8 mg, 1.0-2.0 mg, 1.0-1.8 mg, 1.0-1.6 mg, or 1.2-1.5 mg each time.

In some embodiments, the toll-like receptor 7 agonist or the pharmaceutically acceptable salt thereof is administered once every week and at a dose of 0.2-5.0 mg, 0.4-4.0 mg, 0.5-3.0 mg, 0.6-2.6 mg, 0.8-2.2 mg, 0.8-1.8 mg, 1.0-2.0 mg, 1.0-1.8 mg, 1.0-1.6 mg, or 1.2-1.5 mg each time. In some embodiments, the toll-like receptor 7 agonist or the pharmaceutically acceptable salt thereof is administered once every week and at a dose of 1.2 mg each time. In some embodiments, the toll-like receptor 7 agonist or the pharmaceutically acceptable salt thereof is administered once every week and at a dose of 1.5 mg each time.

In some embodiments, the anti-PD-L1 antibody may be administered once every week, once every two weeks, once every three weeks, or once every four weeks.

In some embodiments, the anti-PD-L1 antibody is administered at a dose of 0.01-40 mg/kg, 0.1-30 mg/kg, 0.1-20 mg/kg, 0.1-15 mg/kg, 0.1-10 mg/kg, 1-15 mg/kg, 1-20 mg/kg, 1-3 mg/kg, 3-10 mg/kg, 3-15 mg/kg, 3-20 mg/kg, 3-30 mg/kg, 10-20 mg/kg, or 15-20 mg/kg each time. In some embodiments, the anti-PD-L1 antibody is administered at a dose of 60 mg-2400 mg, 90 mg-1800 mg, 120 mg-1500 mg, 200 mg-800 mg, 200 mg-600 mg, 300 mg-900 mg, 600 mg-900 mg, 300 mg-1200 mg, 600 mg-1200 mg, or 900 mg-1200 mg each time. In some embodiments, the anti-PD-L1 antibody is administered at a dose of 400 mg each time.

In some embodiments, the anti-PD-L1 antibody may be administered once every three weeks and at a dose of 200 mg-800 mg each time. In some embodiments, the anti-PD-L1 antibody may be administered once every three weeks and at a dose of 400 mg each time.

In some embodiments, entecavir or the pharmaceutically acceptable salt or solvate thereof may be administered once every day, twice every day, or once every two days.

In some embodiments, entecavir or the pharmaceutically acceptable salt or solvate thereof is administered at a dose of 0.005 mg to 5.0 mg each time, preferably a dose of 0.01 mg, 0.05 mg, 0.1 mg, 0.2 mg, 0.3 mg, 0.4 mg, 0.5 mg, 0.6 mg, 0.7 mg, 0.8 mg, 0.9 mg, 1.0 mg, 1.1 mg, 1.2 mg, 1.3 mg, 1.4 mg, 1.5 mg, 1.6 mg, 1.7 mg, 1.8 mg, 1.9 mg, 2.0 mg, 2.1 mg, 2.2 mg, 2.3 mg, 2.4 mg, 2.5 mg, 2.6 mg, 2.7 mg, 2.8 mg, 2.9 mg, 3.0 mg, 3.1 mg, 3.2 mg, 3.3 mg, 3.4 mg, 3.5 mg, 3.6 mg, 3.7 mg, 3.8 mg, 3.9 mg, 4.0 mg, 4.1 mg, 4.2 mg, 4.3 mg, 4.4 mg, 4.5 mg, 4.6 mg, 4.7 mg, 4.8 mg, 4.9 mg, 5.0 mg, or a range formed by any of the values. In some embodiments, entecavir or the pharmaceutically acceptable salt or solvate thereof is administered at a dose of 0.05 mg-5.0 mg, 0.10 mg-2.0 mg, 0.25 mg-2.0 mg, or 0.5 mg-1.0 mg each time.

In some embodiments, entecavir or the pharmaceutically acceptable salt or solvate thereof may be administered once every day and at a dose of 0.10 mg-2.0 mg each time. In some embodiments, entecavir or the pharmaceutically acceptable salt or solvate thereof may be administered once every day and at a dose of 0.5 mg each time.

In some embodiments, the toll-like receptor 7 agonist or the pharmaceutically acceptable salt thereof is administered once every week and at a dose of 1.2 mg or 1.5 mg each time; the anti-PD-L1 antibody is administered once every three weeks and at a dose of 400 mg each time.

In some embodiments, the toll-like receptor 7 agonist or the pharmaceutically acceptable salt thereof is administered once every week and at a dose of 1.2 mg or 1.5 mg each time; the anti-PD-L1 antibody is administered once every three weeks and at a dose of 400 mg each time; entecavir or the pharmaceutically acceptable salt or solvate thereof is administered once every day and at a dose of 0.5 mg each time.

In some embodiments, the toll-like receptor 7 agonist or the pharmaceutically acceptable salt thereof and the anti-PD-L1 antibody are prevented from being administered on the same day.

### Individual in Need Thereof

In some embodiments, the individual in need thereof has a hepatitis B virus infection.

In some embodiments, the individual in need thereof is a hepatitis B patient. In some embodiments, the individual in need thereof is a chronic hepatitis B patient.

In some embodiments, the individual in need thereof has been positive for serum HBsAg for 6 consecutive months or more, and positive or negative for HBeAg.

In some embodiments, the individual in need thereof has been positive for serum HBsAg for 6 consecutive months or more, and positive for HBeAg.

In some embodiments, the individual in need thereof has been positive for serum HBsAg for 6 consecutive months or more, and negative for HBeAg.

In some embodiments, the individual in need thereof is selected from the group consisting of a treatment-naive individual and a treatment-experienced individual.

In some embodiments, the individual in need thereof is selected from a treatment-naive individual who has not previously received any antiviral treatment (total treatment cycle < 12 weeks), or has previously received any nucleoside (nucleotide) analog treatment but the treatment has been stopped for ≥6 months, or has previously received an interferon treatment (including PEGylated or non-PEGylated interferons) but the treatment has been stopped for ≥12 months.

In some embodiments, the individual in need thereof meets the following criteria:
1) positive for serum HBsAg for 6 consecutive months or more;
2) treatment-naive, i.e.,
   has not previously received any antiviral treatment (total treatment cycle < 12 weeks), or has previously received any nucleoside (nucleotide) analog treatment but the treatment has been stopped for ≥6 months, or has previously received an interferon treatment (including PEGylated or non-PEGylated interferons) but the treatment has been stopped for ≥12 months;
3) HBV DNA > 20000 IU/mL for HBeAg-positive patients or HBV DNA > 2000 IU/mL for HBeAg-negative patients;
4) 1×ULN ≤ ALT ≤ 10×ULN.

In some embodiments, the individual in need thereof is selected from a treatment-experienced individual who has consecutively taken any nucleoside (nucleotide) analog or received an interferon treatment (including PEGylated or non-PEGylated interferons) and stuck to a treatment regimen for ≥12 weeks (no interferon treatment for at least 6 months prior to enrollment).

In some embodiments, the individual in need thereof meets the following criteria:
1) positive for serum HBsAg for 6 consecutive months or more;
2) treatment-experienced, i.e.,
   has consecutively taken any nucleoside (nucleotide) analog or received an interferon treatment (including PEGylated or non-PEGylated interferons) and stuck to a treatment regimen for ≥12 weeks (no interferon treatment for at least 6 months prior to receiving the pharmaceutical combination of the present application);
3) HBV DNA < 100 IU/mL 6 months prior to screening period; HBV DNA < 100 IU/mL during screening period;
4) 250 ICT/mL ≤ quantitative HBsAg ≤ 5000 IU/mL.

In some embodiments, the screening period is the 4 weeks prior to the day of receiving the pharmaceutical combination of the present application.

### Definitions and Explanations

Unless otherwise stated, the following terms used in the present application shall have the following meanings. A certain term, unless otherwise specifically defined, should not be considered uncertain or unclear, but construed according to its common meaning in the field.

In the present application, unless otherwise stated, the terms "comprise", "comprises", and "comprising", or equivalents thereof are open-ended statements and mean that elements, components, and steps that are not specified may be included in addition to those listed.

Unless otherwise specified, terms in the singular shall be deemed to include the plural and vice versa. Unless otherwise specified, the word "a" or "an" refers to "at least one". Unless otherwise stated, "or" is used to mean "and/or".

All patents, patent applications, and other identified publications are explicitly incorporated by reference in the present application for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the common knowledge in the art.

As used herein, the dose is based on the weight of the free form of the compound, the free form of the compound referring to the non-salt and non-solvate form of the compound.

As used herein, the term "combination" refers to a combination of two or more active ingredients (administered as the respective active ingredients themselves, or as their respective derivatives like pharmaceutically acceptable salts or esters, solvates, prodrugs, or compositions) that are administered simultaneously or sequentially. The terms "medicinal combination " and "pharmaceutical combination" are used interchangeably in the present application.

As used herein, the term "antibody" refers to a binding protein having at least one antigen-binding domain. The antibody and the fragment thereof of the present application can be an intact antibody or any fragment thereof. Thus, the antibody and the fragment thereof of the present application include a monoclonal antibody or a fragment thereof and an antibody variant or a fragment thereof, as well as an immunoconjugate. Examples of the antibody fragment include a Fab fragment, a Fab' fragment, a F(ab')₂ fragment, an Fv fragment, an isolated CDR region, a single-chain Fv molecule (scFv), an Fd fragment, and other antibody fragments known in the art. The antibody and the fragment thereof may also include a recombinant polypeptide, a fusion protein, and a bispecific antibody. The anti-PD-L1 antibody and the fragment thereof disclosed by the present application can be of the IgG1, IgG2, IgG3, or IgG4 isotype. The term "isotype" refers to the class of antibodies encoded by the heavy chain constant region gene. In one embodiment, the anti-PD-L1 antibody and the fragment thereof disclosed by the present application are of the IgG1 or IgG4 isotype. The anti-PD-L1 antibody and the fragment thereof of the present invention can be derived from any species, including but not limited to, mouse, rat, rabbit, non-human primate, llama, and human. The anti-PD-L1 antibody and the fragment thereof can be a chimeric antibody, a humanized antibody or an intact human antibody. In one embodiment, the anti-PD-L1 antibody is an antibody produced by a hybridoma cell line derived from a mouse. Thus, in one embodiment, the anti-PD-L1 antibody is a murine antibody. In another embodiment, the anti-PD-L1 antibody is a chimeric antibody. In another embodiment, the chimeric antibody is a mouse-human chimeric antibody. In another embodiment, the antibody is a humanized antibody. In another embodiment, the antibody is derived from a murine antibody and is humanized.

The present application provides an isolated antibody or a fragment thereof that binds to PD-L1, wherein the anti-PD-L1 antibody or the fragment thereof can be produced by a hybridoma selected from the group consisting of the hybridomas designated 13C5 and 5G11 disclosed by WO2016022630 or CN107001463. In some embodiments, the anti-PD-L1 antibody or the fragment thereof described in the present invention can be produced by a hybridoma as follows: e.g., hybridomas 13C5 and 5G11, and any hybridoma or cell line capable of producing the antibody or the fragment thereof disclosed by the present application.

The antibody or the antigen-binding fragment thereof disclosed by the present application is specific for PD-L1. In one embodiment, the antibody or the fragment thereof is specific for PD-L1. In one embodiment, the antibody or the fragment thereof provided by the present application binds to human or non-human primate PD-L1 but does not bind to PD-L1 from any other mammal. In another embodiment, the antibody or the fragment thereof does not bind to mouse PD-L1. The terms "human PD-L1", "hPD-L1", "huPD-L1", and the like are used interchangeably in the present application and refer to human PD-L1 and variants or isotypes of human PD-L1. "Specific", "specificity", and "specifically" refer to that the antibody or the fragment thereof binds to PD-L1 with greater affinity than any other target.

The term "treat", "treating" or "treatment" usually refers to acquiring needed pharmacological effect and/or physiological effect. The effect partially or fully stabilizes or cures the disease and/or a side effect of the disease, and can be therapeutic. As used herein, "treat", "treating", and "treatment" encompass any treatment of a disease in a patient, including: (a) inhibiting a symptom of the disease, i.e., blocking the progression of the disease; or (b) alleviating a symptom of the disease, i.e., causing remission of the disease or the symptom.

The term "effective amount" refers to an amount of the compound of the present application for (i) treating a specific disease, condition, or disorder; (ii) alleviating, relieving, or eliminating one or more symptoms of a specific disease, condition, or disorder, or (iii) delaying onset of one or more symptoms of a specific disease, condition, or disorder described in the present application. The amount of active substance (e.g., the antibody or compound of the present application) constituting the "therapeutically effective amount" may vary according to factors such as the disease state, age, sex, and weight of the individual, and the ability of a therapeutic agent or a combination of therapeutic agents to elicit a desired response in the individual. The effective amount may also be determined routinely by those skilled in the art in accordance with their knowledge and the present disclosure.

The terms "administer", "administration", and "administering" refer to physically introducing the composition comprising a therapeutic agent to an individual using any of a variety of methods and delivery systems known to those skilled in the art. Routes of administration of the anti-PD-L1 antibody include intravenous, intramuscular, subcutaneous, intraperitoneal, spinal, or other parenteral routes, e.g., by injection or infusion.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" includes salts formed by compounds comprising basic radicals and free acids and salts formed by compounds comprising acidic radicals and free bases, e.g., hydrochloride, hydrobromide, nitrate, sulfate, phosphate, formate, acetate, trifluoroacetate, fumarate, oxalate, maleate, citrate, succinate, mesylate, benzenesulfonate, *p*-methylbenzenesulfonate, sodium salt, potassium salt, ammonium salt, or amino acid salt, preferably, hydrochloride, hydrobromide, sulfate, formate, acetate, trifluoroacetate, fumarate, maleate, mesylate, p-methylbenzenesulfonate, sodium salt, potassium salt, ammonium salt, or amino acid salt.

In the present application, the terms "subject", "patient", and "individual" are used interchangeably. In some embodiments, the term "subject", "patient", or "individual" refers to a mammal. In some embodiments, the subject, patient, or individual is a mouse. In some embodiments, the subject, patient, or individual is a human.

The term "single dose" refers to the smallest unit of packaging containing a certain quantity of pharmaceutical product; for example, in a box of seven capsules, each capsule is a single dose; or a vial of injection is a single dose. In the present application, the terms "single dose" and "unit dose" have the same meaning and are used interchangeably.

The term "multiple doses" consists of multiple single doses.

In the present application, "average daily dose" refers to the ratio of the total dose to the number of days in a cycle. For example, if the compound of formula **I** is administered once every week and at a dose of 1.2 mg each time, the average daily dose is 1.2 mg/7 days.

The term "fixed combination" refers to the simultaneous administration of active ingredients (e.g., the compound of formula **I** or the anti-PD-L1 antibody or entecavir) to an individual at a fixed total dose or in a fixed dose ratio, or in the form of a single entity, pharmaceutical composition, or formulation. In some embodiments, the active ingredients are present, for example, in one tablet, one capsule, one injection, or one sachet.

The term "non-fixed combination" refers to the simultaneous, parallel, or sequential (without specific time limitation) administration of two or more active ingredients as independent entities (e.g., pharmaceutical compositions or pharmaceutical formulations) to an individual, wherein the active ingredients administered to the individual reach therapeutically effective amounts. Examples of the non-fixed combination can include cocktail therapy, e.g., administration of 2, 3, or more active ingredients. In a non-fixed combination, each active ingredient can be packaged, sold or administered as a fully independent pharmaceutical composition. The "non-fixed combination" further includes combined use of "fixed combinations", or a "fixed combination" and an independent substance of any one or more active ingredients.

The term "pharmaceutical composition" refers to a mixture consisting of one or more of the active ingredients or pharmaceutical combinations thereof of the present application and a pharmaceutically acceptable auxiliary material. The pharmaceutical composition is intended to facilitate the administration of the compound or the pharmaceutical combination thereof of the present application to a subject.

The term "pharmaceutically acceptable auxiliary material" refers to those which do not have a significant irritating effect on an organism and do not impair the biological activity and properties of the active ingredients. Suitable auxiliary materials are well known to those skilled in the art, for example, carbohydrate, wax, water-soluble and/or water-swellable polymers, hydrophilic or hydrophobic material, gelatin, oil, solvent, or water.

The pharmaceutical composition of the present application can be prepared by combining the active ingredient of the present application with a suitable pharmaceutically acceptable auxiliary material and can be formulated, for example, into a solid, semisolid, liquid, or gaseous formulation such as tablet, pill, capsule, powder, granule, ointment, emulsion, suspension, suppository, injection, inhalant, gel, microsphere, and aerosol, etc.

The pharmaceutical composition of the present application can be manufactured using methods well known in the art, such as conventional mixing, dissolving, granulating, dragee-making, levigating, emulsifying, and lyophilizing.

As used herein, the compound of formula **I** or the pharmaceutically acceptable salt thereof may be administered by any applicable route and method, e.g., oral administration or parenteral (e.g., intravenous) administration. The therapeutically effective amount of the compound of formula **I** or the pharmaceutically acceptable salt thereof includes, but is not limited to, from about 0.0001 to 20 mg/kg/d, e.g., from 0.001 to 10 mg/kg/d. The dosing frequency (e.g., once, twice, or more times daily) of the compound of formula **I** or the pharmaceutically acceptable salt thereof can be determined according to the conditions of the patient individuals, including the severity of the disease, the response to the disease, any treatment-related toxicity, and the age and health of a patient. Administration can be intermittent, e.g., in a period of several days, the subject receives a daily dosage of the compound of formula **I** or the pharmaceutically acceptable salt thereof, and in the following period of several days or more days, the patient does not receive the daily dosage of the compound of formula **I** or the pharmaceutically acceptable salt thereof.

Entecavir or the pharmaceutically acceptable salt or solvate thereof can be administered by various routes including, but not limited to, oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular, and intrathecal administrations. In some specific embodiments, the drug is administered orally. The amount of entecavir administered can be determined according to the severity of the disease, the response of the disease, any treatment-related toxicity, and the age and health of a patient. For example, a daily dose of entecavir can be 0.005 mg-10.0 mg. Entecavir can be administered once or more times daily. In some embodiments, entecavir is administered once daily in the form of an oral solid formulation.

In the present application, the term "nucleoside (nucleotide) analog" includes, but is not limited to, entecavir or a pharmaceutically acceptable salt or solvate thereof, or tenofovir or a pharmaceutically acceptable prodrug thereof or a pharmaceutically acceptable salt thereof. Entecavir or the pharmaceutically acceptable salt or solvate thereof is selected from the group consisting of entecavir, entecavir monomaleate, entecavir monohydrate, and entecavir monomaleate monohydrate. Tenofovir or the pharmaceutically acceptable prodrug thereof or the pharmaceutically acceptable salt thereof is selected from the group consisting of tenofovir, tenofovir alafenamide fumarate, tenofovir disoproxil fumarate, tenofovir disoproxil orotate, tenofovir disoproxil hemidisulfonate, tenofovir disoproxil phosphate, tenofovir disoproxil succinate, tenofovir disoproxil aspartate, and tenofovir amibufenamide fumarate.

The term "about" shall be understood to include a range of three standard deviations from a mean value or a standard tolerance range in a specific field. In some embodiments, "about" shall be understood as a variation not exceeding 0.5. "About" modifies all listed values thereafter. For example, "about 1, 2 and 3" represents "about 1", "about 2" and "about 3".

### Administration

The description below does not limit the administration of the pharmaceutical combination of the present application.

The active ingredients in the pharmaceutical combination of the present application can be formulated separately, or some or all of the active ingredients are co-formulated. In one embodiment, the medicinal combination of the present application can be formulated into a pharmaceutical composition which is suitable for a single dose or multiple doses.

The active ingredients in the pharmaceutical combination of the present application can be administered separately, or some or all of the active ingredients are co-administered. The active ingredients in the pharmaceutical combination of the present application can be administered in a substantially asynchronous manner, or some or all of the active ingredients are administered in a substantially synchronous manner.

The active ingredients in the pharmaceutical combination of the present application can be administered independently, or some or all of the active ingredients are co-administered by various proper routes, including, but not limited to, oral administration or parenteral administration (intravenous, intramuscular, topical, or subcutaneous routes). In some embodiments, the active ingredients in the pharmaceutical combination of the present application can be administered independently, or some or all of the active ingredients are co-administered by means of oral administration or injection, e.g., intravenous injection or intraperitoneal injection.

The active ingredients in the pharmaceutical combination of the present application can be in independent suitable dosage forms, or some or all of the active ingredients are co-formulated in a suitable dosage form, including, but not limited to, tablet, lozenge, pill, capsule (for example, hard capsule, soft capsule, enteric capsule, and microcapsule), elixir, granule, syrup, injection (intramuscular, intravenous, and intraperitoneal), powder, emulsion, suspension, solution, dispersion, and dosage forms of sustained-release formulations for oral or non-oral administration.

The active ingredients in the pharmaceutical combination of the present application can be each independently formulated, or some or all of the ingredients are co-formulated with a pharmaceutically acceptable auxiliary material (e.g., a carrier and/or an excipient).

The medicinal combination of the present application may further comprise an additional therapeutic agent. In one embodiment, the additional therapeutic agent can be a therapeutic agent known in the art for treating a hepatitis B virus infection.

### Technical Effects

The pharmaceutical combination of the present application can significantly reduce the HBsAg level in serum or other HBV indicators. In addition, the pharmaceutical combination of the present application demonstrates a significant enhancement compared to monotherapies. These indicate that the pharmaceutical combination of the present application has good pharmaceutical value.

### DETAILED DESCRIPTION

For clarity, the present application is further described with the following examples, which are, however, not intended to limit the scope of the present application. All reagents used in the present application are commercially available and can be used without further purification.

**Example 1:** Combination of Compound of Formula **I,** Anti-PD-L1 Antibody, and Entecavir for Treatment of Hepatitis B

### 1. Test compounds

The compound of formula **I:** made into 0.2 mg or 0.5 mg tablets for later use. The preparation method of the tablets can be found in WO2020216274.

The anti-PD-L1 antibody: having a heavy chain amino acid sequence set forth in SEQ ID NO: 17 and a light chain amino acid sequence set forth in SEQ ID NO: 18. The anti-PD-L1 antibody was made into a 100 mg/10 mL injection for later use.

Entecavir: commercially available entecavir tablets, 0.5 mg/tablet. Commercially available entecavir tablets include, but are not limited to, entecavir dispersible tablets (Runzhong^{®}).

### 2. Enrolled subjects

The subjects should meet all of the following criteria:
1) Male patients or non-pregnant, non-lactating female patients aged 18-65 years (inclusive);
2) Treatment-naive or treatment-experienced chronic hepatitis B (CHB) patients, positive for serum HBsAg for 6 consecutive months or more and positive or negative for HBeAg;
3) The treatment-naive patient is defined as a patient who has not previously received any antiviral treatment (total treatment cycle < 12 weeks), or has previously received any nucleoside (nucleotide) analog treatment but the treatment has been stopped for ≥6 months, or has previously received an interferon treatment (including PEGylated or non-PEGylated interferons) but the treatment has been stopped for ≥12 months;
4) The treatment-experienced patient is defined as a patient who has consecutively taken any nucleoside (nucleotide) analog or received an interferon treatment (including PEGylated or non-PEGylated interferons) and stuck to a treatment regimen for ≥12 weeks prior to enrollment screening (but the interferon treatment has to have been stopped for at least 6 months prior to enrollment);
5) For treatment-naive patients: 1×ULN ≤ ALT ≤ 10×ULN, HBV DNA > 20000 IU/mL for HBeAg-positive patients or HBV DNA > 2000 IU/mL for HBeAg-negative patients;
   For treatment-experienced patients: HBV DNA < 100 IU/mL 6 months prior to the screening period; HBV DNA < 100 IU/mL during the screening period, and 250 IU/mL ≤ quantitative HBsAg ≤ 5000 IU/mL;
6) Electrocardiograph (ECG) results are normal.

### 3. Dosage and administration

The compound of formula **I:** orally administered at 1.2 mg/dose or 1.5 mg/dose, once every week.

The anti-PD-L1 antibody: 400 mg/dose, diluted to 250 mL with normal saline, infused over 60 (±10) min, and intravenously infused once every 21 days.

Entecavir: orally administered at 0.5 mg/dose, once every day.

### 4. Efficacy indicators

Serum HBsAg, HBeAg, HBV DNA, etc.

### 5. Results

**Table 1. Information about some enrolled patients**

| Patient No. | HBeAg | Administration Cycle | History of previous therapy | Administration regimen |
|---|---|---|---|---|
| 01001 | Negative | W48 | Administered entecavir and Wuling pills for ≥12 consecutive weeks | Compound of formula **I:** orally administered at 1.2 mg/dose, once every week; |
| | | | | Entecavir: orally administered at 0.5 mg/dose, once every day. |
| 01005 | Positive | W48 | Administered tenofovir for ≥12 consecutive weeks | Compound of formula **I:** orally administered at 1.2 mg/dose, once every week; |
| | | | | Entecavir: orally administered at 0.5 mg/dose, once every day. |
| 01014 | Positive | W36 | Administered entecavir for ≥12 consecutive weeks | Compound of formula **I:** orally administered at 1.5 mg/dose, once every week; |
| | | | | Entecavir: orally administered at 0.5 mg/dose, once every day. |
| 01022 | Negative | W24 | Administered entecavir for ≥12 | Compound of formula **I:** orally administered at 1.2 mg/dose, once every week; |
| | | | consecutive weeks | Entecavir: orally administered at 0.5 mg/dose, once every day. |
| 01023 | Negative | W24 | Administered entecavir for ≥12 consecutive weeks | Compound of formula **I:** orally administered at 1.2 mg/dose, once every week; |
| | | | | Entecavir: orally administered at 0.5 mg/dose, once every day. |
| 01031 | Negative | W18 | Administered entecavir for ≥12 consecutive weeks | Compound of formula **I:** orally administered at 1.5 mg/dose, once every week; Entecavir: orally administered at 0.5 mg/dose, once every day. |
| 01002 | Negative | W48 | Administered entecavir for ≥12 consecutive weeks | Compound of formula **I:** orally administered at 1.2 mg/dose, once every week; Entecavir: orally administered at 0.5 mg/dose, once every day; |
| | | | | Anti-PD-L1 antibody: intravenously infused at 400 mg/dose, once every 21 days. |
| 01009 | Negative | W48 | Administered entecavir for ≥12 consecutive weeks | Compound of formula **I:** orally administered at 1.2 mg/dose, once every week; Entecavir: orally administered at 0.5 mg/dose, once every day; |
| | | | | Anti-PD-L1 antibody: intravenously infused at 400 mg/dose, once every 21 days. |
| 01015 | Negative | W36 | Administered entecavir for ≥12 consecutive weeks | Compound of formula **I:** orally administered at 1.2 mg/dose, once every week; Entecavir: orally administered at 0.5 mg/dose, once every day; |
| | | | | Anti-PD-L1 antibody: intravenously infused at 400 mg/dose, once every 21 days. |

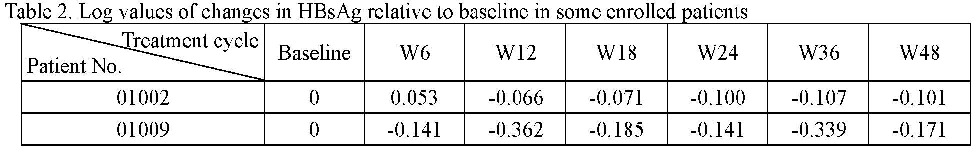

## Claims

1. A pharmaceutical combination comprising a toll-like receptor 7 agonist or a pharmaceutically acceptable salt thereof and an anti-PD-L1 antibody, wherein the anti-PD-L1 antibody comprises the following amino acid sequences: a heavy chain CDR1 region having at least 80% homology with an amino acid sequence set forth in SEQ ID NO: 1 or SEQ ID NO: 4; a heavy chain CDR2 region having at least 80% homology with an amino acid sequence set forth in SEQ ID NO: 2 or SEQ ID NO: 5; a heavy chain CDR3 region having at least 80% homology with an amino acid sequence set forth in SEQ ID NO: 3 or SEQ ID NO: 6; a light chain CDR1 region having at least 80% homology with an amino acid sequence set forth in SEQ ID NO: 7 or SEQ ID NO: 10; a light chain CDR2 region having at least 80% homology with an amino acid sequence set forth in SEQ ID NO: 8 or SEQ ID NO: 11; and a light chain CDR3 region having at least 80% homology with an amino acid sequence set forth in SEQ ID NO: 9 or SEQ ID NO: 12.

2. The pharmaceutical combination according to claim 1, wherein the toll-like receptor 7 agonist or the pharmaceutically acceptable salt thereof is selected from a compound of formula **I** or a pharmaceutical salt thereof:

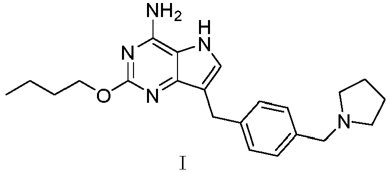

3. The pharmaceutical combination according to claim 1 or 2, wherein the toll-like receptor 7 agonist or the pharmaceutically acceptable salt thereof is in the form of a pharmaceutical composition; optionally, the pharmaceutical composition of the toll-like receptor 7 agonist or the pharmaceutically acceptable salt thereof is selected from a pharmaceutical composition in a single dose of 0.01 mg-10 mg.

4. The pharmaceutical combination according to any one of claims 1-3, wherein the toll-like receptor 7 agonist or the pharmaceutically acceptable salt thereof is administered thrice every day, twice every day, once every day, once every two days, once every three days, once every four days, once every five days, once every six days, once every week, once every two weeks, or once every three weeks.

5. The pharmaceutical combination according to any one of claims 1-4, wherein the toll-like receptor 7 agonist or the pharmaceutically acceptable salt thereof is administered at a dose of 0.1-10.0 mg each time.

6. The pharmaceutical combination according to any one of claims 1-5, wherein the anti-PD-L1 antibody comprises the following amino acid sequences: a heavy chain variable region having at least 80% homology with an amino acid sequence set forth in SEQ ID NO: 13 or SEQ ID NO: 14; and a light chain variable region having at least 80% homology with an amino acid sequence set forth in SEQ ID NO: 15 or SEQ ID NO: 16.

7. The pharmaceutical combination according to any one of claims 1-6, wherein the anti-PD-L1 antibody comprises the following amino acid sequences: a heavy chain amino acid sequence set forth in SEQ ID NO: 17; and a light chain amino acid sequence set forth in SEQ ID NO: 18.

8. The pharmaceutical combination according to any one of claims 1-7, wherein the anti-PD-L1 antibody is in the form of a pharmaceutical composition; optionally, the pharmaceutical composition of the anti-PD-L1 antibody is selected from a pharmaceutical composition in a single dose of 200 mg-600 mg.

9. The pharmaceutical combination according to any one of claims 1-8, wherein the anti-PD-L1 antibody is administered once every week, once every two weeks, once every three weeks, or once every four weeks.

10. The pharmaceutical combination according to any one of claims 1-9, wherein the anti-PD-L1 antibody is administered at a dose of 60 mg-2400 mg each time.

11. The pharmaceutical combination according to any one of claims 1-10, wherein a weight ratio of the toll-like receptor 7 agonist or the pharmaceutically acceptable salt thereof to the anti-PD-L1 antibody is 1:(10-200).

12. The pharmaceutical combination according to any one of claims 1-11, further comprising entecavir or a pharmaceutically acceptable salt or solvate thereof.

13. The pharmaceutical combination according to claim 12, wherein the entecavir or the pharmaceutically acceptable salt or solvate thereof is administered once every day, twice every day, or once every two days.

14. The pharmaceutical combination according to claim 12 or 13, wherein the entecavir or the pharmaceutically acceptable salt or solvate thereof is administered at a dose of 0.005 mg-5.0 mg each time.

15. The pharmaceutical combination according to any one of claims 12-14, wherein a weight ratio of the toll-like receptor 7 agonist or the pharmaceutically acceptable salt thereof to the anti-PD-L1 antibody to the entecavir or the pharmaceutically acceptable salt or solvate thereof is 1:(10-200):(1-10).

16. The pharmaceutical combination according to any one of claims 1-15 for use in treating a hepatitis B virus infection.

17. Use of the pharmaceutical combination according to any one of claims 1-15 for preparing a medicament for treating a hepatitis B virus infection.

18. A kit for use in treating a hepatitis B virus infection, wherein comprising the pharmaceutical combination according to any one of claims 1-15 and instructions for combined use for treating the hepatitis B virus infection.

19. A method for treating a hepatitis B virus infection comprising administering to an individual in need thereof a therapeutically effective amount of the pharmaceutical combination according to any one of claims 1-15.
